(19) Europäisches Patentamt
European Patent Office
Office européen des brevets

(11) Publication number: **0 091 565**
**B1**

(12) # EUROPEAN PATENT SPECIFICATION

(45) Date of publication of patent specification: **23.04.86**

(21) Application number: **83102458.3**

(22) Date of filing: **12.03.83**

(51) Int. Cl.[4]: **C 07 D 217/04,**
C 07 D 211/70,
C 07 D 405/04,
C 07 D 471/04,
C 07 D 211/78, A 61 K 31/475

(54) **Process for the manufacture of isoquinoline diones.**

(30) Priority: **29.03.82 US 363332**
**29.03.82 US 363333**

(43) Date of publication of application:
**19.10.83 Bulletin 83/42**

(45) Publication of the grant of the patent:
**23.04.86 Bulletin 86/17**

(84) Designated Contracting States:
**AT BE CH DE FR GB IT LI NL**

(56) References cited:
**EP-A-0 010 661**
**EP-A-0 035 244**

**CHEMICAL ABSTRACTS, vol. 92, no. 13, 31st March 1980, page 641. no. 110798y, Columbus, Ohio, USA I.A. KOZELLO et al.: "Improvement in the method of preparing arecoline based on acetaldehyde"**

**CHEMICAL ABSTRACTS, vol. 82, no. 23, 8th December 1975, page 437, no. 193038p, Columbus, Ohio, USA R.V. STEVENS: "Acid-catalyzed rearrangement of cyclobutylimines. New synthesis of tetrahydropyridines"**

(73) Proprietor: **F. HOFFMANN-LA ROCHE & CO. Aktiengesellschaft**
**CH-4002 Basel (CH)**

(72) Inventor: **Coffen, David Llewellyn**
**270 Ridgewood Avenue**
**Glen Ridge, N.J. (US)**
Inventor: **Hengartner, Urs Oskar**
**28 Binzenstrasse**
**CH-4058 Basle (CH)**

(74) Representative: **Bertschinger, Christoph, Dr. et al**
**Grenzacherstrasse 124 P.O. Box 3255**
**CH-4002 Basel (CH)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The present invention relates to a facile process for the manufacture of bicyclic diketones, namely hexahydro-6,8(1H,7H)-isoquinoline diones of the general formula

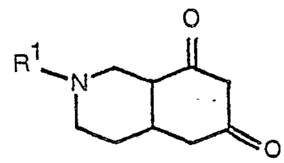

I

wherein $R^1$ is lower alkyl or aryl-lower alkyl.

As used herein, the term "lower alkyl" denotes a straight or branched chain saturated hydrocarbon group of 1 to 7 carbon atoms, for example, methyl, ethyl, propyl, isopropyl, butyl, pentyl, heptyl and the like. The term "aryl" denotes phenyl or substituted phenyl. The substituents on the phenyl moiety may comprise one or more halogen, lower alkyl and the like. Exemplary of aryl-lower alkyl are benzyl, methylbenzyl and the like. The term "halogen" means fluorine, chlorine, bromine and iodine. The term "lower alkoxy" denotes lower alkyl ether groups, for example, methoxy, ethoxy and the like.

Heretofore, compounds of formula I have been obtained in rather poor yields from 3,4-dihydro-1H-6,8-dimethoxy-2-methylisoquinoline hydrochloride by Birch reduction and hydrolysis, cf. EP—A—10 661 and EP—A—35 244.

In accordance with the present invention, the compounds of formula I can be manufactured by a process which comprises reacting a compound of the general formula

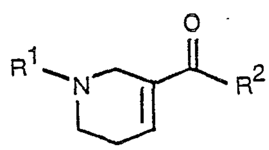

II

with a compound of the general formula

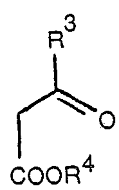

III

wherein $R^1$ is as described above, one of $R^2$ and $R^3$ is methyl and the other is lower alkoxy and $R^4$ is lower alkyl, in the presence of an alkali metal alkoxide and treating sequentially the reaction mixture obtained with a base and then with an acid.

More specifically, the process of the present invention comprises, on the one hand, the reaction of a compound of the formula II, wherein $R^2$ is methyl, i.e. a compound of the general formula

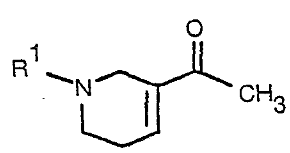

IIa

wherein $R^1$ is as described above, with a di-lower alkyl malonate, for example, dimethyl malonate, diethyl malonate and the like, and, on the other hand, the reaction of a compound of the formula II, wherein $R^2$ is lower alkoxy, i.e. a compound of the general formula

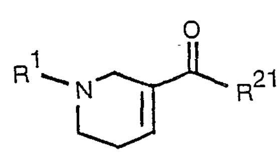

IIb

wherein $R^1$ is as described above and $R^{21}$ is lower alkoxy, with a lower alkyl acetoacetate, for example, methyl acetoacetate and the like.

Both these embodiments of the process according to the present invention are performed in a suitable solvent, for example, a lower alkanol, such as, methanol, ethanol, propanol, t-butanol and the like, and in the presence of an alkali metal alkoxide, such as, sodium ethoxide, potassium methoxide, potassium t-butoxide and the like. At this stage of the process, a carbanion derived from the compound of formula III undergoes Michael addition to the α,β-unsaturated ketone or ester function of the compound of formula II. The adduct thus formed undergoes cyclization under the reaction conditions employed.

Thereafter, the reaction mixture is treated sequentially with a base, for example, an alkali metal hydroxide, such as, sodium hydroxide, potassium hydroxide and the like, and an acid, for example, an inorganic acid, such as, hydrochloric acid, hydrobromic acid and the like. This treatment effects hydrolysis and decarboxylation, respectively, of the redundant ester function introduced with the compound of formula III to yield a hexahydro-6,8(1H,7H)-isoquinolinedione of formula I.

This reaction is carried out at a temperature in the range of from about room temperature to about the reflux temperature of the reaction mixture. The desired hexahydro-6,8(1H,7H)-isoquinolinedione can be recovered and purified utilizing conventional methods, for example, ion exchange chromatography and the like. The compounds of formula I are known compounds. Exemplary of the compounds of formula I are hexahydro-2-methyl-6,8(1H,7H)-isoquinolinedione, hexahydro-2-benzyl-6,8(1H,7H)-isoquinolinedione, hexahydro-2-ethyl-6,8(1H,7H)-isoquinolinedione and the like.

The compounds of formulas II and III used as starting materials are known compounds or can be prepared in accordance with known procedures. Exemplary of the compounds of formula II are 1-(1,2,5,6-tetrahydro-1-methyl-3-pyridyl)ethanone (arecolone), 1-(1,2,5,6-tetrahydro-1-ethyl-3-pyridinyl)ethanone, 1-(1,2,5,6-tetrahydro-1-benzyl-3-pyridinyl)ethanone and methyl 1,2,5,6-tetrahydro-1-methyl-nicotinate (arecoline).

Alternatively, the compounds of formula IIa can be manufactured according to the process described hereinafter in Reaction Scheme I. This process is novel and also forms part of the present invention.

REACTION SCHEME I

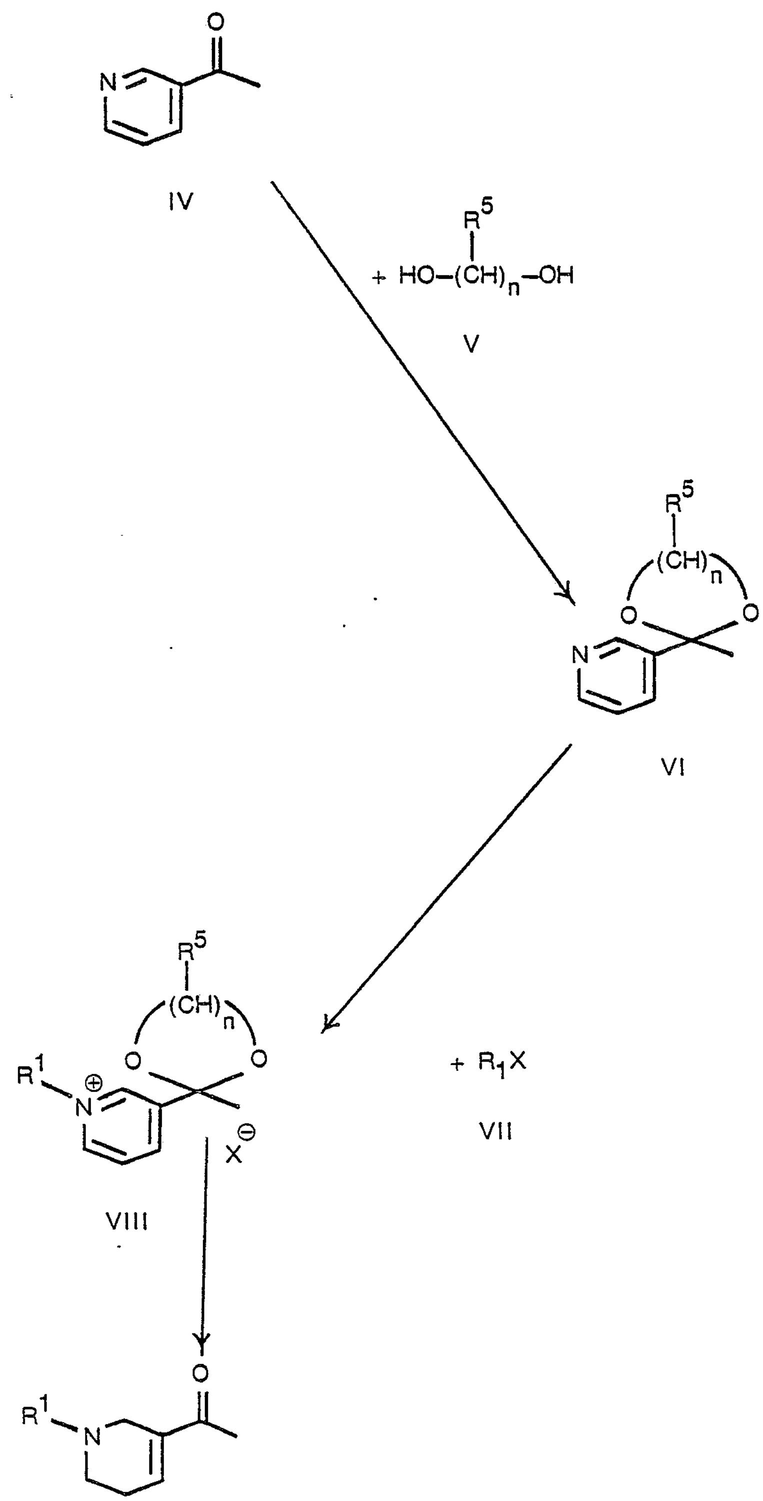

IIa

wherein $R^1$ is as previously described, $R^5$ is hydrogen or methyl, X is halogen and n is 2 to 4.

In accordance with Reaction Scheme I, 3-acetyl pyridine of formula IV is treated with a glycol of formula V in the presence of boron trifluoride etherate and the like, in an aromatic hydrocarbon solvent, for example, xylene, benzene and the like, to yield a compound of formula VI, for example, 3-(2-methyl-1,3-dioxolan-2-yl)pyridine. If desired, an obtained compound of formula VI can be used in the subsequent reaction without isolation and purification. Alternatively, a compound of formula VI can be recovered by distillation and the like.

Thereafter, a compound of formula VI is reacted with a lower alkyl or aryl-lower alkyl halide of formula VII to yield a pyridinium compound of formula VIII. Exemplary of the halides of formula VII are benzyl chloride, methyl iodide, ethyl bromide and the like. The reaction is carried out in an aromatic hydrocarbon solvent, for example, xylene, benzene and the like, at a temperature in the range of room temperature to the reflux temperature. The compound of formula VIII usually precipitates from the reaction mixture and can be recovered by filtration.

A compound of formula VIII is then, in a first step, treated with a reducing agent, for example, sodium borohydride, and an alkali metal alkoxide such as sodium methoxide and the like. The reduction is carried out in an alkanol, for example, methanol, ethanol and the like, at the reflux temperature of the reaction mixture. In a second step, the reaction mixture is treated with an acid, for example, a hydrohalic acid such as hydrochloric acid and the like, and finally made alkaline with a base such as potassium carbonate and the like, to yield a compound of formula IIa. If desired, a compound of formula IIa is converted into an acid addition salt for convenience of storage and handling. The conversion is carried out utilizing a hydrohalic acid such as a hydrobromic acid, hydrochloric acid and the like. The compounds of formula IIa can be recovered by extractive isolation, distillation and the like. The acid addition salts of compounds of formula IIa are generally solids and can be recovered by filtration.

The compounds of formula I are useful as intermediates in the manufacture of, for example, 4,4a,5,6,7,8,8a,9-octahydro-4a,8a-trans-1H-pyrrolo[2,3-g]isoquinolin-4-ones of the general formula

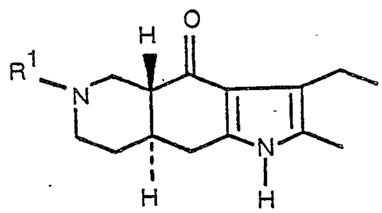

IX

wherein $R^1$ is as described above, which in turn are useful as antipsychotic agents, for example in the treatment of schizophrenia.

More specifically, a hexahydro-6,8(1H,7H)-isoquinolinedione of formula I is reacted with 2-oximino-3-pentanone, utilizing the conditions of a Knorr pyrrole synthesis, to yield the correspondingly substituted 4,4a,5,6,7,8,8a,9-octahydro-4a,8a-trans-1H-pyrrolo[2,3-g]isoquinolin-4-one of formula IX.

The reaction is carried out, for instance, in the presence of zinc dust and an acid, for example, acetic acid, at a temperature in the range of from about room temperature to about the reflux temperature of the reaction mixture. The compound of formula I can be used without isolation and purification. The desired 4,4a,5,6,7,8,8a,9-octahydro-4a,8a-trans-1H-pyrrolo[2,3-g]isoquinolin-4-one of formula IX is recovered utilizing conventional methods, for example, by crystallization and the like. The conversion of a compound of formula I to a 4,4a,5,6,7,8,8a,9-octahydro-4a,8a-trans-1H-pyrrolo[2,3-g]isoquinolin-4-one is further described in United States Patent No. 4,260,762, issued April 7, 1981.

The examples which follow further illustrate the invention. All temperatures are in degrees centrigrade unless otherwise stated.

Example 1

Preparation of 1-(1,2,5,6-tetrahydro-1-methyl-3-pyridinyl)ethanone hydrobromide

a) A 3 l three-necked flask equipped with a mechanical stirrer and a Dean Stark water separator with reflux condenser was charged with 228 g of p-toluenesulfonic acid monohydrate and a 1.2 l of toluene. The mixture was stirred and heated at reflux for 30 minutes. A slightly orange solution was formed and 19 ml of water were collected in the separator. The heating mantle was removed and 88.6 g of ethylene glycol was added, followed by the addition of a solution of 121.1 g of 3-acetylpyridine in 150 ml of toluene over a period of 5 minutes. The resulting two phase mixture was vigorously stirred at reflux for 2 hours. An additional 9.2 g of ethylene glycol was added and stirring at reflux was continued for 1 hour (27 ml of water were collected in this 3 hour period). The reaction mixture was cooled quickly to 20° and then poured into a well stirred mixture of 1.0 l of 3N aqueous sodium hydroxide and 500 g of ice. The mixture was stirred for 5 minutes. The aqueous layer was separated and was extracted with 3 × 600 ml portions of toluene. Each portion of toluene extract was washed sequentially with the same 500 ml of brine. The combined toluene extracts were dried over anhydrous potassium carbonate and concentrated on a rotavap. The residual oil was distilled at vacuo, yielding 156.3 g 3-(2-methyl-1,3-dioxolan-2-yl)pyridine as colorless mobile oil, b.p. 73—75°/0.9 mm.

b) A 3 l three-necked flask, equipped with mechanical stirrer, reflux condenser and thermometer was charged under nitrogen with 155 g of 3-(2-methyl-1,3-dioxolan-2-yl)pyridine and 1.2 l of toluene. 272 g of methyl iodide was added. The mixture was stirred and heated at 53° (internal temp.) for 32 hours and then

let stand at room temperature overnight. The precipitate was collected by filtration, washed with 500 ml of toluene, 500 ml of hexane and dried (r.t., 0.1 mm, const. weight) to yield 286.2 g (99.3%) of 1-methyl-3-(2-methyl-1,3-dioxolan-2-yl)pyridinium iodide as light yellow crystals, m.p. 185.5—187° (sinters at 167°).

c) A 5 l three-necked flask, equipped with mechanical stirrer, thermometer and reflux condenser with nitrogen inlet was charged under nitrogen with 285.8 g of 1-methyl-3-(2-methyl-1,3-dioxolan-2-yl)pyridinium iodide and 2.0 l of absolute ethanol. The suspension was stirred while 38.0 g of sodium borohydride was added in small portions over 30 minutes keeping the internal temperature at 25° with an ice bath. At this point all starting material had gone into solution. Then an additional 60 g of sodium borohydride was added in portions over 30 minutes while the internal temperature was kept at 35° with a cold water bath. The mixture was then stirred at reflux for 30 minutes. The heating mantle was removed. 1.0 l of 6N aqueous hydrochloric acid was slowly added from a dropping funnel and the white suspension stirred at reflux for 1 hour. The reflux condenser was replaced by a Claisen distilling head connected to a condenser and an ice-bath cooled receiver. 1.8 l of solvent was distilled at vacuo (180 mm, b.p. 54—60°). Then the reaction mixture was cooled to 20° and stirred while 1.0 l of water was added, followed by portionwise addition of 900 g of anhydrous potassium carbonate ($CO_2$-evolution).

The product, which separated as a brown organic layer, was taken up in 1.0 l of ether and the aqueous layer, containing some crystalline inorganic salt, was extracted with 3/500 ml portions of ether. The combined ether extracts were dried over anhydrous potassium carbonate and concentrated on a rotavap. The residual red brown oil (132 g) was distilled at vacuo to give 114.5 g of 1-(1,2,5,6-tetrahydro-1-methyl-3-pyridinyl)ethanone as light yellow oil, b.p. 58—75°/0.9 mm.

d) 114.5 g of 1-(1,2,5,6-tetrahydro-1-methyl-3-pyridinyl)ethanone was placed in a 2 l beaker and dissolved in 500 ml of absolute ethanol. The solution was stirred (mechanical stirrer) in an icebath, while 150.0 g of 48% aqueous hydrobromic acid was added dropwise from a dropping funnel at such a rate that the temperature did not exceed 25°. The resulting slurry was stirred in the icebath for 30 minutes. The precipitate was collected by filtration, washed with 2 × 175 ml portions of ice cold absolute ethanol and dried in vacuo (0.1 mm, 20°, const. weight) to yield 132.5 g of 1-(1,2,5,6-tetrahydro-1-methyl-3-pyridinyl)-ethanone hydrobromide as white crystals, m.p. 225—226°. The filtrate was concentrated on a rotavap (50°, 15 mm) and the residual paste dissolved in 110 ml of absolute ethanol at reflux. The solution was stirred in an ice bath for 30 minutes. The precipitate collected by filtration, washed with 2 × 60 ml portions of ice cold absolute ethanol and dried (0.1 mm, 20°, const. weight) to yield a second crop of 14.5 g 1-(1,2,5,6-tetrahydro-1-methyl-3-pyridinyl)ethanone hydrobromide as white crystals, m.p. 219—221°.

Example 2

Preparation of 3-ethyl-2,6-dimethyl-4,4a,5,6,7,8,8a,9-octahydro-4a,8a,trans-1H-pyrrolo[2,3-g]isoquinolin-4-one

a) A 1 l beaker was charged with a solution of 144.1 g of 1-(1,2,5,6-tetrahydro-1-methyl-3-pyridinyl)ethanone hydrobromide, in 400 ml of water. Then, 220 g of anhydrous potassium carbonate was added portionwise with stirring; the free base separated as a mobile oil. The mixture was extracted with 500 ml and 2 × 250 ml portions of chloroform.

The combined extracts were dried over anhydrous potassium carbone and concentrated on a rotavap (20 mm, 35°). To remove some remaining chloroform, the residual oil was taken up in 250 ml of ethylacetate and concentrated again on a rotavap leaving 101 g of 1-(1,2,5,6-tetrahydro-1-methyl-3-pyridinyl)ethanone as a yellow oil.

b) A 3 l three-necked flask, equipped with a mechanical stirrer, thermometer and reflux condenser was charged under nitrogen with 750 ml of absolute ethanol. 21.2 g of freshly cut sodium metal was added and the stirred mixture was slowly brought to reflux. After all the sodium had reacted, the solution was cooled to 20°. 119.5 g of diethyl malonate and 0.655 mol of the above prepared 1-(1,2,5,6-tetrahydro-1-methyl-3-pyridinyl)ethanone (101 g containing approx. 10 g solvent), were added. The solution was stirred at reflux for 5 hours.

The following day 95 g of 85% potassium hydroxide pellets were added and the mixture stirred at reflux for 4 hours. The reflux condenser was replaced by a Claisen distilling head connected to a condenser with a cooled (dry-ice-acetone bath) receiver. 1.2 l of solvent was distilled at vacuo (200 mm) while 1.2 l of water was gradually added from a dropping funnel, keeping the volume of the reaction mixture constant. The solution was cooled below 60° and 400 ml of concentrated hydrochloric acid (12N) was slowly added from a dropping funnel. The resulting clear yellow solution was stirred at reflux for 1 hour. The reactor was again set for distillation (as described above) and the solution was concentrated in vacuo, first at 10 mm (aspirator vacuum), then at 4 mm (pump) leaving a very viscous semisolid paste which contains the hydrochloride salt of hexahydro-2-methyl-6,8(1H,7H)-isoquinolinedione.

c) This material was taken up in 1.4 l of glacial acetic acid at 50° and then cooled to 15°. 69.0 g of 2-oximino-3-pentanone, and 98,1 g of zinc dust were added. The mixture was stirred for 3 minutes at room temperature and then the moderately exothermic Knorr reaction was initiated by heating the flask content to about 45°. Without external heating, the internal temperature rose slowly over 5 minutes to 95°. The mixture was then heated at reflux for 20 minutes. The flask contents were quickly cooled to 20° in an ice bath and 57,5 g of 2-oximino-3-pentanone and 65.5 g of zinc dust were added. The resulting exothermic reaction heated the mixture to 65° (5 minutes), which was then stirred again at reflux for 20 minutes. It was

quickly cooled to 20° and a third portion of 57.5 g of 2-oximino-3-pentanone and 65.5 g of zinc dust were added. After the internal temperature leveled at 65°, the reaction mixture was stirred at reflux for 1 hour. Then the reactor was set for distillation (as described above) and the brown solution concentrated at aspirator vacuum to a brown viscous oil (650 ml acetic acid was collected). 1.2 l of water was added, some residual zinc was removed by filtration and the filtrate was placed in an ice cooled 4 l beaker. 1.5 l of concentrated ammonia (29%) was slowly added with stirring. The precipitate was collected on a coarse, sintered glass funnel and washed with 2 × 500 ml portions of water. The wet cake was dissolved in 1.4 l of 1.7N aqueous hydrochloric acid at 70° and the free base was again precipitated by adding 300 ml of concentrated ammonia (29%) to the warm (70°) solution. The cooled reaction mixture was filtered and the filter cake was washed with 3 × 1 l portions of water.

The wet cake (308 g; mp 246—250°) was transferred into 2 l beaker, 1.2 l of acetone was added and the white suspension was stirred at room temperature for 30 minutes. The product was collected by filtration, washed with 500 ml of ether and dried [50°, 2 mm, 4hr. then r.t., 0.1 mm, const. weight] to yield 73.0 g (45%) of crude 3-ethyl-2,6-dimethyl-4,4a,5,6,7,8,8a,9-octahydro-4a,8a-trans-1H-pyrrolo[2,3-g]isoquinolin-4-one as an off white powder, mp 256—259°. This material was dissolved in 1.0 l of ethanol and 400 ml of methylene chloride at reflux. The slightly turbid brown solution was filtered through a bed of Celite which was washed with 150 ml of methylene chloride. The clear filtrate was placed in a 3 l three-necked flask (fitted with mechanical stirrer, thermometer and Claisen distilling head connected to a condenser and a cooled receiver). 700 ml of solvent, mostly methylene chloride, was removed by distillation; toward the end of the distillation the product began to crystallize.

The mixture was allowed to cool to room temperature and was then stirred in an icebath for 2 hours. The precipitate was collected by filtration, washed with 150 ml of cold (−20°) ethanol and dried (r.t., 0.1 mm, const. weight) to yield 63.0 g (39% from 1-(1,2,5,6-tetrahydro-1-methyl-3-pyridinyl)ethanone hydrobromide) of 3-ethyl-2,6-dimethyl-4,4a,5,6,7,8,8a,9-octahydro-4a,8a-trans-1H-pyrrolo[2,3-g]isoquinolin-4-one as slighly pink crystals, mp 262—264°.

### Example 3

Preparation of hexahydro-2-methyl-6,8(1H,7H)-isoquinolinedione

A 250 ml three necked flask, equipped with magnetic stirrer, reflux condenser and nitrogen inlet was charged with 80 ml ethanol. Thereafter, 2.28 g of sodium metal, freshly cut, was added and the mixture stirred till all the sodium had dissolved. Then, 12.33 g of diethylmalonate in 6 ml of ethanol, and 9.74 g of 1-(1,2,5,6-tetrahydro-1-methyl-3-pyridinyl)ethanone (arecolone) in 6 ml of ethanol, were added to the warm solution and the resulting solution was stirred and heated to reflux for 5 hours. Then, 10.2 g of 85% potassium hydroxide pellets were added and the mixture heated to reflux for four hours. During this period a heavy precipitate formed. With the aid of 50 ml water, the mixture was transferred into a 500 ml flask and concentrated at 30° and 20 mm of pressure. The residue was again concentrated from 50 ml of water. It was then taken up in 100 ml water. 45 ml 12N hydrochloric acid were added and the solution was heated to reflux for 2 hours. It was then cooled and concentrated on a rotovap at 40° and 20 mm of pressure.

Purification was effected by:

a) Ion exchange chromatography using 2M aqueous pyridine as eluent on a Dowex AG 50W—X8 column; and

b) Recrystallization from water which afforded 3.87 g of hexahydro-2-methyl-6,8(1H,7H)-isoquinolinedione as colorless, hydroscopic crystals having a melting point of 141—144°.

### Example 4

Preparation of the hydrochloride salt of hexahydro-2-methyl-6,8(1H,7H)-isoquinolinedione.

Three hundred (300) mg of hexahydro-2-methyl-6,8(1H,7H)-isoquinolinedione were dissolved in 6 ml of ethanol with 0.2 ml of concentrated hydrochloric acid. The mixture was stirred at room temperature while 8 ml of anhydrous ether was added slowly. The mixture was then chilled in an ice bath for one hour. The precipitate was filtered, washed with cold ether and dried at room temperature and 0.2 mm of pressure for 3 days to yield 133.1 mg of the hydrochloride salt of hexahydro-2-methyl-6,8(1H,7H)-isoquinolinedione as white crystals having a melting point of 214—217° d.

| | | | | |
|---|---|---|---|---|
| Analysis Calculated | C, 55.17; | H, 7.41; | N, 6.43; | Cl, 16.28 |
| Found | C, 55.10; | H, 7.42; | N, 6.41; | Cl, 16.44 |

### Example 5

Preparation of 3-ethyl-2,6-dimethyl-4,4a,5,6,7,8,8a,9-octahydro-1H-pyrrolo[2,3-g]isoquinolin-4-one hydrochloride

a) Methyl 1,2,5,6-tetrahydro-1-methylnicotinate hydrobromide (Arecoline HBr) weighing 350 g, was dissolved in 600 ml of water. To this solution 50 g of sodium chloride was added and allowed to dissolve. To this solution approximately 400 ml of saturated sodium carbonate solution was added and the mixture extracted four times with (4 ×) 500 ml of ether. The combined ether extracts were dried over sodium sulfate and the ether evaporated under reduced pressure using a 40° water bath. The methyl 1,2,5,6-tetrahydro-1-methylnicotinate was distilled under aspirator vacuum to give 188 g (82%) of a pale yellow liquid with b.p. 85°/20 mm (pressure approximate). The free base was stored in a freezer.

b) Potassium metal weighing 83 g was washed free of oil with petroleum ether, weighed out in petroleum ether, and added to a 3 l one neck flask containing 1800 ml of t-butyl alcohol. The potassium dissolved during several hours (approximately 8) of heating at reflux with magnetic stirring under argon. Thereafer, 140 g of distilled methyl acetoacetate was added to the cooled potassium t-butoxide solution followed by 160 g of methyl 1,2,5,6-tetrahydro-1-methylnicotinate. The resulting red solution was stirred under argon at room temperature for 24 hours and then at reflux for 48 hours. A solution of 300 g of potassium hydroxide in 700 ml of water was added carefully in portions with the condenser set down for distillation. Heating and stirring were continued for approximately 2 hours during which 1750 ml of distillate, wet t-butyl alcohol, was collected between 80—94°.

With continued refluxing in a vertical condenser 800 ml of concentrated hydrochloric acid was added in very small portions. Vigorous spattering and degassing accompanied the initial additions. The condenser was again set down and boiling continued for 1 hour during which an additional 100 ml of distillate was collected. The solution was cooled and concentrated as much as possible on the rotary evaporator using aspirator vacuum. Optional extraction, after cooling, with ether will remove most of the 1,3-dimethyl-2-pyridone by-product at this point.

c) The salt mixture from Example 5 b), in the same flask was taken up into 1800 ml of glacial acetic acid and cooled in an ice bath. Then, 40 g of 2-oximino-3-pentanone and 45 g of zinc dust were added and the cold mixture was stirred for 15 minutes. A mechanical stirrer was used for this step. The mixture was then stirred at reflux for 15 minutes then cooled again in an ice bath. Forty g (40 g) of 2-oximino-3-pentanone and 45 g of zinc dust were added and the mixture stirred 15 minutes while kept cold. It was heated to reflux for 15 minutes, again cooled in ice and a third portion of 40 g of 2-oximino-3-pentanone and 45 g of zinc dust added. After another 15 minutes of stirring while cold, the mixture was heated to reflux again and kept at reflux for 75 minutes. The bath was left at room temperature overnight at this point. Most of the acetic acid was removed using a rotary evaporator and aspirator vacuum. To the residue, there were added 1250 ml of 3N hydrochloric acid and 1050 ml of water to dissolve the residue.

The solution was transferred to a 4 l separatory funnel and washed three times with (3 ×) 1000 ml of ether. The ether layer from each wash was discarded, and 1500 ml of concentrated aqueous ammonia was added to the aqueous layer to make it strongly basic while keeping the zinc salts in solution. The crude product separated as a solid precipitate. It was collected, washed with water, and air-dried overnight to give 33.3 g of light yellow powder. An additional 1.6 g of crude product was extracted from the filtrate and washings as described below to give an overall crude yield of 39.9 g = 15.7%. The aqueous filtrate and washings were extracted three times with (3 ×) 1000 ml of methylene chloride. The residue from the extract after drying with sodium sulfate and evaporation was about 100 ml of brown oil. Volatile byproducts in this fraction were vacuum distilled off at 45—75°/0.05 mm. The distillation residue was taken up in 100 ml of ether and chilled overnight to give, after filtering, washing with ether and air-drying, 1.6 g of additional crude product.

The 38.3 g of dried precipitate was taken up in 500 ml of hot ethanol and filtered through Celite to remove some insoluble material. The Celite cake was washed twice with (2 ×) 100 ml of hot ethanol. The filtered solution was concentrated to about 250 ml by boiling. After chilling overnight in a −40° freezer, the solid was collected, washed with ether and air-dried to give 20.0 g of reddish, crystalline product which is pure by thin layer chromatography. Evaporation of solvent from the filtrate and washings left a dark, semisolid mush. This was triturated with ether, filtered and the solid washed with ether to give 4.7 g of "second crop" material. By combining this material and the 1.6 g fraction with the 20 g of once recrystallized product, 26.2 g of product was recrystallized by dissolving in a boiling solvent mixture of 300 ml of methylene chloride and 200 ml of ethanol. 2 g of charcoal was added and the solution was left for 20 minutes. After filtration through Celite and washing the cake twice with (2 ×) 50 ml of 1:1 methylene chloride-ethanol, the solution was boiled down to about 250 ml during which much of the product crystallized. After chilling in a −40° freezer for 4 hours, the product was collected, washed with ether, and air-dried to give 20.0 g of pink crystals with m.p. 260—5° (some decomp.). Thereafter, 20.0 g of twice recrystallized 3-ethyl-2,6-dimethyl-4,4a,5,6,7,8,8a,9-octahydro-1H-pyrrolo[2,3-g]isoquinolin-4-one was treated with a solution of 10 ml of concentrated hydrochloric acid diluted to 100 ml with ethanol. The resulting mixture was heated on the steam bath giving a clear red solution. This was gradually diluted to 1000 ml with ether, adding small portions until precipitation commenced. The mixture was kept in the −40° freezer for 1 hour and then filtered. The solid was washed twice with ether and air-dried to give 23.04 g of very pale pink powder with m.p. 185—8°. After vacuum drying overnight at 85°, the 3-ethyl-2,6-dimethyl-4,4a,5,6,7,8,8a,9-octahydro-1H-pyrrolo[2,3-g]isoquinolin-4-one hydrochloride weighed 22.3 g (6% based arecoline HBr).

**Claims**

1. A process for the manufacture of bicyclic diketones of the general formula

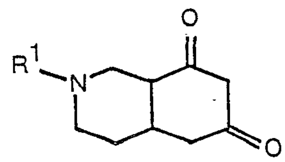

I

wherein $R^1$ is lower alkyl or aryl-lower alkyl, which comprises reacting a compound of the general formula

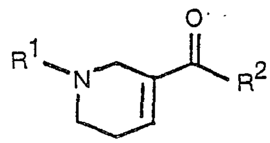

II

with a compound of the general formula

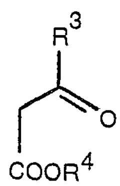

III

wherein $R^1$ is as described above, one of $R^2$ and $R^3$ is methyl and the other is lower alkoxy and $R^4$ is lower alkyl, in the presence of an alkali metal alkoxide and treating sequentially the reaction mixture obtained with a base and then with an acid, the term "lower alkyl" denoting $C_{1-7}$ alkyl and the term "aryl" denoting phenyl or substituted phenyl.

2. A process in accordance with claim 1, wherein the compound of formula II is 1-(1,2,5,6-tetrahydro-1-methyl-3-pyridinyl)ethanone.

3. A process in accordance with claim 2, wherein the compound of formula III is diethyl malonate.

4. A process in accordance with claim 1, wherein the compound of formula II is methyl 1,2,5,6-tetrahydro-1-methylnicotinate.

5. A process in accordance with claim 4, wherein the compound of formula III is methyl acetoacetate.

6. A process in accordance with claim 1, wherein a compound of the general formula

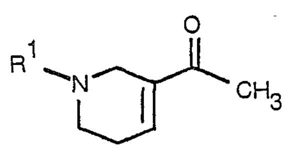

IIa

wherein $R^1$ is as described in claim 1, is used as starting material and wherein said compound of formula IIa is prepared by a process which comprises the steps of

a) reacting 3-acetylpyridine of the formula

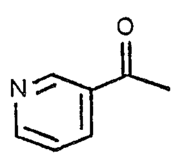

IV

with a glycol of the general formula

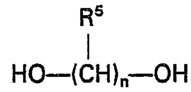

V

wherein $R^5$ is hydrogen or methyl and n is 2 to 4, to yield a compound of the formula

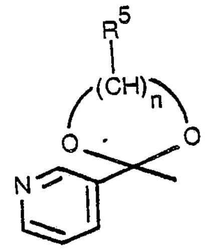

VI

wherein $R^5$ is as described above,

b) treating the reaction product of step a) with a halide of the general formula

$$R^1X \qquad VII$$

wherein $R^1$ is as previously described and X is halogen, to yield a compound of the formula

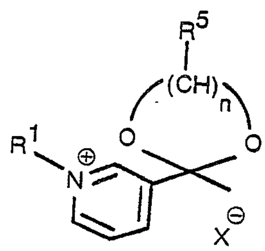

VIII

wherein $R^1$, $R^5$, X and n are as previously described, and

c) treating the reaction product of step b) with a reducing agent and then sequentially with an acid and a base to yield a compound of the formula IIa.

**Patentansprüche**

1. Verfahren zur Herstellung von bicyclischen Diketonen der allgemeinen Formel

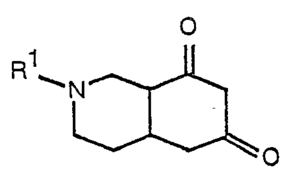

I

worin $R^1$ nieder Alkyl oder Aryl-nieder alkyl bedeutet, dadurch gekennzeichnet, dass man eine Verbindung der allgemeinen Formel

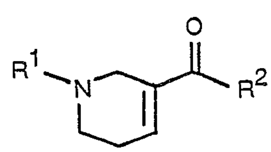

II

mit einer Verbindung der allgemeinen Formel

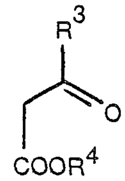

III

worin $R^1$ die oben angegebene Bedeutung besitzt, einer von $R^2$ und $R^3$ Methyl und der andere nieder Alkoxy und $R^4$ nieder Alkyl bedeuten, in Gegenwart eines Alkalimetallalkoxids umsetzt, und das haltene Reaktionsgemisch nacheinander mit einer Base und einer Säure behandelt, wobei der Ausdruck "nieder Alkyl" $C_{1-7}$-Alkyl und der Ausdruck "Aryl" Phenyl oder substituiertes Phenyl bedeuten.

2. Verfahren gemäss Anspruch 1, dadurch gekennzeichnet, dass die Verbindung der Formel II 1-(1,2,5,6-Tetrahydro-1-methyl-3-pyridinyl)ethanon ist.

3. Verfahren gemäss Anspruch 2, dadurch gekennzeichnet, dass die Verbindung der Formel III Diäthylmalonat ist.

4. Verfahren gemäss Anspruch 1, dadurch gekennzeichnet, dass die Verbindung der Formel II Methyl 1,2,5,6-tetrahydro-1-methylnicotinat ist.

5. Verfahren gemäss Anspruch 4, dadurch gekennzeichnet, dass die Verbindung der Formel III Methylacetoacetat ist.

6. Verfahren gemäss Anspruch 1, dadurch gekennzeichnet, dass man eine Verbindung der allgemeinen Formel

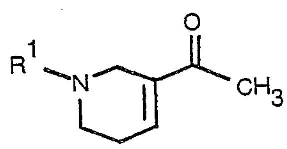

IIa

worin $R^1$ die in Anspruch 1 angegebene Bedeutung besitzt, als Ausgangsmaterial verwendet und dadurch gekennzeichnet, dass die genannte Verbindung der Formel IIa nach einem Verfahren hergestellt worden ist, das die folgenden Reaktionsschritte umfasst:

a) Umsetzen von 3-Acetylpyridin der Formel

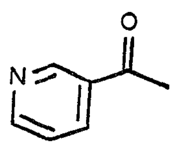

IV

mit einem Glycol der allgemeinen Formel

$$HO{-}(\overset{R^5}{\overset{|}{C}}H)_n{-}OH \qquad V$$

worin $R^5$ Wasserstoff oder Methyl und n 2 bis 4 bedeuten, unter Bildung einer Verbindung der Formel

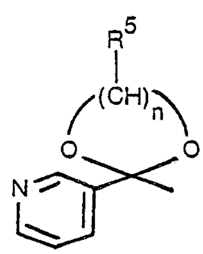

VI

worin $R^5$ die oben angegebene Bedeutung besitzt,

b) Umsetzen des Reaktionsprodukts von Reaktionsschritt a) mit einem Halogenid der allgemeinen Formel

$$R^1X \qquad VII$$

worin $R^1$ die vorstehend angegebene Bedeutung besitzt und X Halogen bedeutet, unter Bildung einer Verbindung der Formel

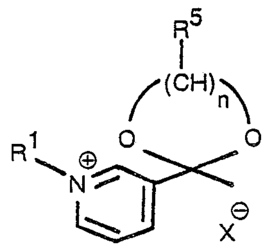

VIII

worin $R^1$, $R^5$, X und n die vorstehend angegebene Bedeutung besitzen, und

c) Behandlung des Reaktionsprodukts aus Reaktionsschritt b) mit einem Reduktionsmittel und danach nacheinander mit einer Säure und einer Base unter Bildung einer Verbindung der Formel IIa.

**Revendications**

1. Procédé de fabrication des dicétones bicycliques de formule générale

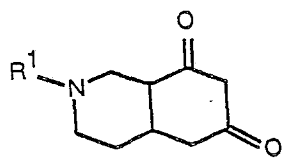

I

dans laquelle $R^1$ est un alkyle inférieur ou un aryl-(alkyle inférieur), qui comprend la réaction d'un composé de formule générale

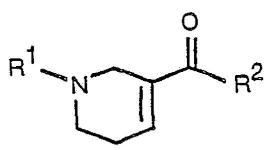

II

avec un composé de formule générale

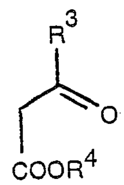

III

dans lesquelles $R^1$ est tel que décrit ci-dessus, l'un de $R^2$ et $R^3$ est le méthyle et l'autre est un alcoxy inférieur et $R^4$ est un alkyle inférieur, en présence d'un alcoxyde de métal alcalin et le traitement séquentiel du mélange réactionnel obtenu avec une base puis avec un acide, le terme "alkyle inférieur" désignant un alkyle en $C_{1-7}$ et le terme "aryle" désignant le phényle ou phényle substitué.

2. Procédé selon la revendication 1, dans lequel le composé de formule II est la 1-(1,2,5,6-tétrahydro-1-méthyl-3-pyridinyl)éthanone.

3. Procédé selon la revendication 2, dans lequel le composé de formule III est le malonate de diéthyle.

4. Procédé selon la revendication 1, dans lequel le composé de formule II est le 1,2,5,6-tétrahydro-1-méthylnicotinate de méthyle.

5. Procédé selon la revendication 4, dans lequel le composé de formule III est l'acétoacétate de méthyle.

6. Procédé selon la revendication 1, dans lequel on utilise comme matériau de départ un composé de formule générale

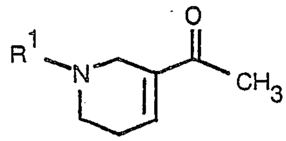

IIa

dans laquelle $R^1$ est tel que décrit dans la revendication 1, et dans lequel le dit composé de formule IIa est préparé par un procédé qui comprend les étapes de

a) réaction de la 3-acétylpyridine de formule

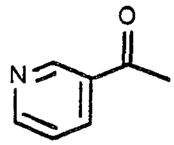

IV

avec un glycol de formule générale

$$\begin{array}{c} R^5 \\ | \\ HO-(CH)_n-OH \end{array} \qquad V$$

dans laquelle $R^5$ est l'hydrogène ou le méthyle et n est 2 à 4, pour donner un composé de formule

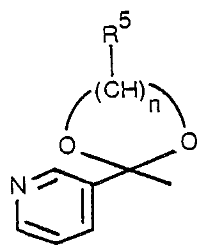

VI

dans laquelle $R^5$ est tel que défini ci-dessus,

b) traitement du produit de réaction de l'étape a) avec un halogénure de formule générale

$$R^1X \qquad VII$$

dans laquelle $R^1$ est tel que décrit précédemment et X est un halogène, pour donner un composé de formule

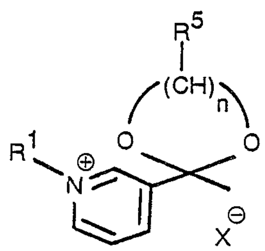

VIII

dans laquelle, $R^1$, $R^5$, X et n sont tels que décrits précédemment, et

c) traitement du produit de réaction de l'étape b) avec un agent réducteur et ensuite séquentiellement avec un acide et une base pour donner un composé de formule IIa.